(19) Europäisches Patentamt
European Patent Office
Office européen des brevets

(11) **EP 4 428 586 A1**

(12) **EUROPÄISCHE PATENTANMELDUNG**

(43) Veröffentlichungstag:
**11.09.2024   Patentblatt 2024/37**

(21) Anmeldenummer: **23160638.5**

(22) Anmeldetag: **08.03.2023**

(51) Internationale Patentklassifikation (IPC):
**G02B 6/04** (2006.01)      **A61B 1/07** (2006.01)

(52) Gemeinsame Patentklassifikation (CPC):
**G02B 6/04; A61B 1/00167; A61B 1/07**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **SCHOTT AG**
**55122 Mainz (DE)**

(72) Erfinder:
• **SCHULTHEIS, Bernd**
**55270 Schwabenheim (DE)**

• **CRAMER, Martin**
**65187 Wiesbaden (DE)**
• **GRIMM, Jonas**
**65307 Bad Schwalbach (DE)**
• **BREY, Fabian**
**60389 Frankfurt a. M. (DE)**
• **KNAUS, Jonas**
**55116 Mainz (DE)**
• **WILLMES, Lothar**
**65375 Oestrich-Winkel (DE)**
• **KRAUS, Sergej**
**64319 Pfungstadt (DE)**

(74) Vertreter: **Schott Corporate IP**
**Hattenbergstraße 10**
**55122 Mainz (DE)**

(54) **FASEROPTISCHER LICHTLEITER MIT VERFORMTEN ENDEN**

(57)   Die Erfindung betrifft einen faseroptischen Lichtleiter (1), beinhaltend eine Mehrzahl von Lichtleitfasern (10), welche als flexible Faserbündel mit im Wesentlichen konstantem Durchmesser $d_1$ (11) zusammengefasst sind und an mindestens einem Ende des faseroptischen Lichtleiters (1) zumindest in Teilbereichen seiner Länge vollumfänglich von einem Hüllmaterial (20) umschlossen ist und die Lichtleitfasern (10) miteinander und vorzugsweise auch mit dem Hüllmaterial (20) verschmolzen sind, wodurch ein starrer Bereich mit einer Längsachse (16) gebildet ist, wobei im Bereich des Hüllmaterials (20) der faseroptische Lichtleiter (1) mittels Einwirkung von Druck und Temperatur aus seiner Längsachse (16) heraus gebogen und/ oder hinsichtlich seiner Querschnittsgeometrie umgeformt ist. Als weitere Ausführungsvarianten kann der faseroptische Lichtleiter (1) auch zusätzlich im starren Bereich verjüngt ausgeführt sein. Die Erfindung betrifft weiterhin ein Verfahren zur Herstellung derartiger Lichtleiter (1).

Fig. 1

EP 4 428 586 A1

**Beschreibung**

Die Erfindung betrifft einen faseroptischen Lichtleiter, beinhaltend eine Mehrzahl von Lichtleitfasern, welche als flexible Faserbündel mit im Wesentlichen konstantem Durchmesser $d_1$ zusammengefasst beziehungsweise zusammenfassbar sind und an mindestens einem Ende des faseroptischen Lichtleiters zumindest in Teilbereichen seiner Länge vollumfänglich von einem Hüllmaterial umschlossen ist und die Lichtleitfasern dort miteinander und vorzugsweise auch mit dem Hüllmaterial verschmolzen sind und einen starren Abschnitt bilden.

Ein Hauptanwendungsgebiet faseroptischer Lichtleiter sind beispielsweise Lichtleiter für Endoskope für insbesondere medizinische oder auch industrielle Anwendungen. Bei diesen besteht die Notwendigkeit, dass der Lichtleiter einerseits mit einem ersten Ende (proximales Ende) beziehungsweise, dessen Endfläche an einer Lichtquelle angekoppelt werden und andererseits mit einem zweiten Ende (distales Ende) in den zu untersuchenden Körper eingeführt werden soll, insbesondere um dorthin Licht zu leiten oder Licht von dort aufzunehmen. Lichtquellen mit hoher Lichtleistung werden allgemein gewünscht. Je größer die Eintrittsfläche und somit der Durchmesser des Lichtleiters ist, desto besser oder einfacher ist er an eine Lichtquelle effizient anzukoppeln, aber je größer sein Durchmesser ist, desto schlechter ist er in den zu untersuchenden Körper einzuführen. Aus diesem Grunde weisen einige endoskopische Lichtleiter mehrere Teilbereiche auf und umfassen beispielsweise einen ersten Teilbereich mit weitestgehend konstantem größeren Durchmesser $d_1$ auf, an den sich in der Regel ein weiterer Teilbereich aus einem zusätzlichen Lichtleiter bestehender kegelförmiger Verjüngungsbereich anschließt, in dem sich der Durchmesser des Lichtleiters auf einen kleineren Durchmesser $d_2$ reduziert. An den Verjüngungsbereich kann dann als ein weiterer Teilbereich ein weiterer Lichtleiter mit dem kleineren Durchmesser $d_2$ angeschlossen werden, der in den zu untersuchenden Körper oder Gegenstand einführbar ist.

In einigen Ausführungen von derartigen Endoskopen können insbesondere an dessen distalem Ende auch weitere Komponenten, wie beispielsweise zur Bildleitung oder Bildaufnahme, beispielsweise Kameras oder Kamerachips, sowie Lichtquellen umfasst sein. Hier ist mittels der faseroptischen Beleuchtung, insbesondere zu Bildaufnahme eine geeignete Ausleuchtung zu gewährleisten. Dies kann durch die Anordnung an oder um derartige weitere Komponenten und/oder angepasste Geometrie der Lichtleiter erreicht werden.

Der Verjüngungsbereich wird in der bisher bekannten Herstellungsweise durch das Umformen eines separaten Faserbündels zu einem Kegel erzeugt und anschließend mit dem Lichtleiter mit einem konstanten Durchmesser verbunden, beispielsweise durch Verschmelzen. Dadurch entsteht eine Grenzfläche zwischen Lichtleiter und Verjüngungsbereich, an welcher die Faserstruktur des gesamten Lichtleiters unterbrochen ist und im Betriebszustand durch Teilreflektion durch den Lichtleiter geführtes beziehungsweise geleitetes Licht verlorengeht, so dass die Gesamttransmission des Lichtleiters reduziert wird.

Ferner liegen Lichtleiter üblicherweise als ungeordnetes Faserbündel vor, bei dem die Lichtleitfasern aus Kern-Mantel-Fasern bestehen. Kern-Mantel-Fasern sind Fasern, die aus einem lichtleitenden Kern bestehen, der entlang der Faserachse vollumfänglich von einem Mantel aus einem Material mit einem niedrigeren Brechungsindex als das Material des Kerns umschlossen ist. Durch Totalreflektion an der Grenzfläche zwischen Kern und Mantel wird die Lichtleitung im Faserkern ermöglicht. Durch das Anbringen des separaten Verjüngungsbereichs kann nicht gewährleistet werden, dass ein Faserkern aus dem Lichtleiterbereich mit dem konstanten Durchmesser mit einem Faserkern aus dem Verjüngungsbereich verbunden wird. Vielmehr entsteht an der Grenzfläche zwischen dem ersten Teilbereich und dem Verjüngungsbereich eine statistische Verteilung aus in den Fasermantel einkoppelnden und zumindest teilweise in den Kern einkoppelnden Fasern. Weil das Licht aber nicht oder nur erheblich schlechter im Fasermantel geleitet werden kann, führt auch dies zu einer Verringerung der Gesamttransmission des Lichtleiters.

DE 100 13 482 C2 beschreibt ein Verfahren zur Herstellung eines faseroptischen Lichtleiters, der ein Bündel lichtleitender Fasern umfasst, das vor dem Einbringen in eine Fassung in einer Glashülse bei geeigneter Temperatur kollabiert wird, wobei die Glashülse nach dem Kollabieren der Fasern und vor dem Einbringen der Fasern in die Fassung von dem Bündel der Fasern entfernt wird, wobei weiterhin vor dem Kollabieren eine Zwischenschicht zwischen dem Bündel der Fasern und die Glashülse eingebracht wird, eine verbesserte Entfernung der Glashülse von dem Bündel der Fasern nach dem Kollabieren zu ermöglichen. Das Verfahren sieht dabei vor, dass als Zwischenschicht eine Schicht eines pulverförmigen, feinkörnigen oder pastenartigen Trennmittels eingebracht wird, dessen Schmelzpunkt über der Erweichungstemperatur der Fasern und der der Glashülse liegt, wobei vor dem Einbringen der Zwischenschicht die Innenseite der Glashülse zumindest abschnittsweise aufgeraut wird.

DE 197 03 515 C1 beschreibt einen faseroptischen Lichtleiter, aus einem Faserbündel bestehend, der an mindestens einem seiner Enden in eine vorgegebene Fassung einführbar ist und eine optisch wirksame Bearbeitung erfährt. Dabei ist vorgesehen, dass das in die vorgegebene Fassung einführbare Ende vor seiner Einführung in einer Glashülse kollabiert und dass die Glashülse vor der Einführung des Endes in die vorgegebene Fassung beseitigt worden ist.

In beiden Fällen wird die Glashülse temporär aufgebracht und nach Bearbeitung mit teils aufwendigen Verfahren vor dem Einbau insbesondere der Enden in eine Ferrule beziehungsweise Fassung oder Hülse entfernt.

Die Anmelderin hat einen Lichtleiter und ein Verfahren zu dessen Herstellung entwickelt, bei dem die Glashülse auf dem Faserbündel, insbesondere auch nach dem Verjüngen des Querschnittes verbleibt und als festen Verbund zur Stabilisierung des faseroptischen Lichtleiters beiträgt, also der Lichtleiter im Bereich des Hüllmaterials starr ausgebildet ist. Beschädigungen, wie beispielsweise die Beschädigung der äußeren Randlage der Lichtleitfasern, beim weiteren Prozessieren, insbesondere auch beim Entfernen der Glashülse, können damit ausgeschlossen werden.

Mit diesem Ansatz können insbesondere kreisrunde Querschnitte an insbesondere einer verjüngten Endfläche beziehungsweise Endabschnitt mit einer Verjüngung, dargestellt werden.

Im Hinblick auf neuere Anforderungen, beispielsweise bei speziellen Endoskopen unter Berücksichtigung von dem zur Verfügung stehendem Bauraum am distalen Ende eines Endoskops, ist es Aufgabe der Erfindung, einen Lichtleiter bereitzustellen, der neben runden auch unrunde Endflächen aufweist, beziehungsweise von einer rein kegelförmigen, im Wesentlichen uniformen Verjüngung des Lichtleiters, das heißt beispielsweise mit im Wesentlichen gleichbleibender Querschnittsgeometrie, abweichende Geometrien zu realisieren und neben einem damit starren, geformten Bereich auch unterbrechungsfrei, einen eigenen, kontinuierlich an den starren Bereich anschließenden flexiblen Bereich umfasst.

Offenbarung der Erfindung:

Die Aufgabe der Erfindung wird gelöst durch einen faseroptischen Lichtleiter mit den Merkmalen gemäß Anspruch 1.

Es wird ein faseroptischer Lichtleiter bereitgestellt, welcher einen flexiblen Bereich der Länge $l_1$ mit im Wesentlichen konstantem Durchmesser $d_1$ an seiner ursprünglichen Endfläche aufweist, einen weiteren Bereich mit einem Hüllmaterial umfasst, wobei im Bereich des Hüllmaterials der, dort dann späterhin starre, faseroptische Lichtleiter mittels beispielsweise Einwirkung von Druck und Temperatur aus seiner Längsachse heraus, also beispielsweise mit zumindest einer Biegung in zumindest einem Teilbereich des Bereiches mit Hüllmaterial, gebogen, verbogen oder abgewinkelt ist und/ oder in diesem starren Bereich hinsichtlich seines Querschnitts beziehungsweise seiner Querschnittsgeometrie, umgeformt ist. Damit sind faseroptische 3D-verformte Lichtleiter realisierbar, die sich insbesondere in Endoskope verbauen lassen, bei denen der zur Verfügung stehende Bauraum in der Regel begrenzt ist, beziehungsweise Kollision mit anderen Komponenten im Endoskop zu vermeiden sind.

Mit anderen Worten liegt so ein faseroptischer Lichtleiter vor, der einen flexiblen Bereich aufweist, an dessen Endfläche dieser an eine Lichtquelle angeschlossen ist beziehungsweise anschließbar oder zuordenbar ist. Der Lichtleiter weist in seinem vom Hüllmaterial umgebenen, somit starren, Bereich zumindest eine Biegung oder Abwinkelung aus seiner ursprünglichen Längsachse beziehungsweise aus der Richtung dieser Längsachse heraus auf. Der faseroptische Lichtleiter ist also verformt, insbesondere zumindest im starren Bereich einmal abgewinkelt beziehungsweise gebogen, und/ oder der faseroptische Lichtleiter weist in diesem starren Bereich, zumindest einen Bereich mit veränderter beziehungsweise umgeformter Querschnittsgeometrie auf. Die Querschnittsgeometrie des faseroptischen Lichtleiters ist also beispielsweise von einer zunächst, ursprünglich, runden Form in eine unrunde Form, beispielsweise ovale oder auch polygonale Form, überführt beziehungsweise umgeformt. Der mithin starre, gebogene und/oder in seiner Querschnittsgeometrie umgeformte, Bereich des faseroptischen Lichtleiter bildet in der Regel das dem proximalen Ende gegenüberliegende, distale Ende des Lichtleiters aus, aus dem das im Betriebszustand am proximalen Ende eingekoppelte Licht ausgekoppelt und bereitgestellt wird. Unter der Verformung aus der Längsachse heraus werden also insbesondere auch eine Biegung oder Abwinkelung verstanden und unter der Umformung der Querschnittsgeometrie eben deren geometrische Veränderung beziehungsweise Veränderung der Querschnittsform.

Als Längsachse beziehungsweise ursprüngliche Längsachse wird dabei die Achse angesehen, die durch den mit einem Hüllmaterial umgebenen, somit starren, Bereich vorgegeben wird beziehungsweise vorgebbar ist, bevor dieser zumindest einmal gebogen oder abgewinkelt vorliegt.

Eine Variante sieht einen faseroptischen Lichtleiter vor, welcher einen flexiblen Bereich der Länge $l_1$ mit im Wesentlichen konstantem Durchmesser $d_1$ an seiner ursprünglichen Endfläche aufweist und zumindest einen sich daran anschließenden Verjüngungsbereich der Länge $l_2$, in dem sich der Durchmesser des Lichtleiters von dem Durchmesser $d_1$ auf den Durchmesser $d_2$ verringert. Dabei ist der Verjüngungsbereich zumindest in Teilbereichen seiner Länge vollumfänglich von dem Hüllmaterial umschlossen und die Faserstruktur setzt sich ohne Unterbrechung von dem Bereich mit dem im Wesentlichen konstanten Durchmesser in den Verjüngungsbereich fort, wobei der Verjüngungsbereich eine verjüngte Endfläche aufweist, in der die Lichtleitfasern miteinander und vorzugsweise auch mit dem Hüllmaterial verschmolzen sind. Die Länge $l_2$ kann dabei auch einen Bereich umfassen, welcher, sich der eigentlichen Verjüngung anschließend, mit zunächst, also insbesondere vor einer weiteren Verformung, Biegung oder Abwinkelung, gleichbleibendem Querschnitt beziehungsweise Durchmesser d2 und Querschnittsgeometrie verläuft. Auch kann im Bereich des Hüllmaterials der faseroptische Lichtleiter mittels Einwirkung von Druck und Temperatur aus seiner Längsachse heraus gebogen und/ oder hinsichtlich seiner Querschnittsgeometrie, insbesondere auch im Bereich der Länge $l_2$, umgeformt sein.

Mit anderen Worten liegt so ein faseroptischer Lichtleiter vor, der einen flexiblen Bereich aufweist an dessen Endfläche (proximales Ende) dieser an eine Lichtquelle angeschlossen beziehungsweise anschließbar oder zuordenbar ist und der im seinem vom Hüllmaterial umgebenen, somit starren, Bereich einen Verjüngungsbereich der Länge $l_2$ umfasst, in dem der ursprüngliche Durchmesser $d_1$ auf einen Durchmesser $d_2$ verjüngt ist und zumindest eine Biegung oder Abwinkelung aus seiner ursprünglichen Längsachse beziehungsweise aus der Richtung dieser Längsachse heraus aufweist. Der faseroptische Lichtleiter ist also zumindest im starren Bereich einmal geformt abgewinkelt beziehungsweise gebogen, und/ oder der faseroptische Lichtleiter weist in diesem starren Bereich, zumindest einen Bereich mit veränderter, umgeformter Querschnittsgeometrie auf. Die Querschnittsgeometrie des faseroptischen Lichtleiters ist also beispielsweise von einer zunächst, ursprünglich, runden Form in eine unrunde Form, beispielsweise ovale oder auch polygonale Form, überführt.

Der mithin starre, verjüngte, gebogene und/oder in seiner Querschnittsgeometrie umgeformte, Bereich des faseroptischen Lichtleiter bildet in der Regel das dem proximalen Ende gegenüberliegende, distale Ende des Lichtleiters aus, aus dem das im Betriebszustand am proximalen Ende eingekoppelte Licht ausgekoppelt und bereitgestellt wird.

Als Längsachse beziehungsweise ursprüngliche Längsachse wird dabei die Achse angesehen, die durch den mit einem Hüllmaterial umgebenen, somit starren, Bereich vorgegeben wird beziehungsweise vorgebbar ist, bevor dieser zumindest einmal gebogen oder abgewinkelt vorliegt.

Zum einen kann damit das Licht im Nahfeld, also in kleinem Abstand zwischen Lichtaustrittsfläche des Lichtleiters und dem Objekt (zum Beispiel Gewebeoberfläche) konzentriert werden.

Zum anderen vergrößert sich bei faseroptischen Bauteilen der Akzeptanzwinkel und damit auch der mögliche Abstrahlwinkel beim Verjüngen derartiger Bauteile (beziehungsweise beim Tapern). Dieser Effekt ist umso stärker, je größer der Unterschied der Durchmesser $d_1$ und $d_2$ ist. Maßgeblich ist hierbei die Erhaltung der sogenannten "Etendue" beziehungsweise des Lichtleitwertes, welche die maximal übertragene oder übertragbare Strahlung charakterisiert. Diese Erhaltungsgröße beschreibt die geometrische Fähigkeit eines optischen Systems, Licht hindurchzulassen. Ihr Wert ergibt sich aus dem Produkt aus Öffnungsgröße des optischen Systems und dem durchstrahlten Raumwinkel. Bei faseroptischen Lichtleitern ist insbesondere die numerische Apertur beziehungsweise der Akzeptanzwinkel, unter dem Licht noch im Lichtleiter geführt werden kann, bestimmend für einen möglichen Raumwinkel.

Vereinfacht lässt sich dieser Sachverhalt mit der folgenden Beziehung beschreiben:

$$NA_1 * D_1 = NA_2 * D_2 \qquad\qquad (1)$$

und

$$NA_1 = \sin(\alpha_1) \qquad\qquad (2a)$$

$$NA_2 = \sin(\alpha_2) \qquad\qquad (2b)$$

mit dem ursprünglichen Faserdurchmesser $D_1$, dem verjüngten Faserdurchmesser $D_2$, der numerischen Apertur $NA_1$ der ursprünglichen Faser und der numerischen Apertur $NA_2$ der verjüngten Faser, wobei $\alpha_1$ beziehungsweise $\alpha_2$ der Akzeptanzwinkel zur numerischen Apertur $NA_1$ beziehungsweise $NA_2$ ist. So kann eine Aufweitung des Akzeptanzwinkels und somit eine breitere Ausleuchtung erzielt werden. Dies hat Vorteile, insbesondere, wenn weitwinkelige Kamera-Module in einem Endoskop verbaut sind. So kann beispielsweise mit einer Lichtleitfaser, welche eine NA von 0,68 (entspricht einem doppelten Akzeptanzwinkel von etwa 85°) aufweist durch eine Reduktion des Faserdurchmessers von zum Beispiel $D_1$ = 70 $\mu$m auf ca. $D_2$ = 60 $\mu$m, entsprechend einer Verjüngung des Durchmessers des Lichtleiters von dem Durchmesser $d_1$ auf den Durchmesser $d_2$ von zum Beispiel 4 mm ($d_1$) auf ca. 3,4 mm ($d_2$), eine NA-Aufweitung auf ca. 0,79 erzielt werden was einer Aufweitung des halben Akzeptanzwinkels von 42,5° auf etwa 52,2° beziehungsweise 104,4° als doppelter Akzeptanz entspricht. Damit kann das Abstrahlverhältnis des Lichtleiters dem Sichtwinkel beispielsweise einer Kamera angepasst werden.

An dieser Stelle sei darauf hingewiesen, dass die Verjüngung, das heißt das Verhältnis von $D_1/D_2$ bezogen auf den Durchmesser der Lichtleitfasern beziehungsweise $d_1/d_2$ bezogen auf das Faserbündel nicht beliebig groß sein kann, um den zuvor beschriebenen Effekt der Strahlaufweitung zu erzielen. So lassen sich bei Lichtleitfasern, deren Ausgangs-NA beispielsweise 0,57 beträgt, was einem doppelten Akzeptanzwinkel von etwa 70° entspricht, nur ein Verhältnis $D_1/D_2$ beziehungsweise $d_1/d_2$ von etwas über 1,75 erzielen. Bei einer anderen Lichtleitfaser mit höherer Ausgangs-NA, beispielsweise NA = 0,68, welche einem doppelten Akzeptanzwinkel von etwa 85° entspricht, lässt sich ein max. Verjüngungsverhältnis von 1,47 erzielen. Weiterhin muss beachtet werden, dass beim Verjüngen der Lichtleitfasern auch die Manteldicke der Lichtleitfasern sich entsprechend verringern, so dass bei Wandstärken im Bereich vom 1- bis 2-fachen der maximal zu übertragenden Lichtwellenlänge (zum Beispiel maximal 1 $\mu$m) infolge von quantenme-

chanischen Effekten die Totalreflexion im Übergangsbereich Kern zu Mantel verlorengeht und der Lichtleiter Licht unkontrolliert zur Seite abstrahlt und damit seine lichtleitenden Eigenschaften verliert. Sinnvolle Verjüngungsverhältnisse liegen daher im Bereich bis maximal 1,5. bis maximal 1,7, abhängig von der NA der lichtleitenden Fasern.

In einer bevorzugten Ausführungsvariante kann vorgesehen sein, dass die Querschnittsfläche der Endfläche am Lichtleiter oder die verjüngte Endfläche des verjüngten Lichtleiters im Wesentlichen unrund, also zum Beispiel oval, D-förmig oder nierenförmig oder auch polygonal verformt bzw. umgeformt ist. Damit kann beispielsweise eine um einen in der Regel quadratischen Kamera-Chip angeordnete Lichtaustrittsfläche, insbesondere am distalen Ende, realisiert werden, um eine homogene Ausleuchtung am distalen Ende eines Endoskops zu gewährleisten. Durch eine gezielte Umformung der distalen Endfläche aus der an sich kreisrunden Form kann der zur Verfügung stehende Gesamtdurchmesser am distalen Ende eines Endoskopes damit optimal ausgenutzt werden. Dies trifft weiterhin zu, wenn zudem noch Arbeitskanäle im oder am Endoskop vorgesehen sind, um beispielsweise minimalinvasive Instrumente darin einzuführen oder anzuordnen und zu nutzen

In einigen Ausführungsformen können entweder Kunststofflichtleitfasern (POF) oder auch Quarz-basierte Lichtleiter zum Einsatz kommen. Besonders vorteilhaft ist es, wenn die Lichtleitfasern Kern-Mantel-Fasern aus Glas, insbesondere aus Multikomponentengläsern, sind und das Hüllmaterial aus einem Glas besteht und in Form einer Glashülse ausgestaltet ist, dessen thermischer Ausdehnungskoeffizient sich höchstens um 50%, bevorzugt höchsten um 30% von dem thermischen Ausdehnungskoeffizienten des Kernglases der Lichtleitfasern unterscheidet. Bei sehr guten optischen Eigenschaften bietet dieser Ansatz Vorteile bei der Verarbeitung und späteren Handhabung gegenüber Kunststofffasern oder Quarzfasern.

Hinsichtlich einer Reduzierung von Streulicht, welches im Betriebszustand, das heißt bei Einkopplung von Licht in den Lichtleiter, aus diesem, insbesondere zu dessen Seite hin, also seitlich aus dem Lichtleiter, austreten kann, hat es sich in weiteren Ausführungsformen als vorteilhaft erwiesen, wenn das Hüllmaterial ein farbiges Glas beziehungsweise eingefärbtes Glas, insbesondere braunes oder schwarzes Glas, umfasst oder daraus besteht und/ oder ein Glas mit einer Licht-absorbierenden Beschichtung umfasst oder daraus besteht. Derartige eingefärbte Gläser können durch geeignete Farbgläser, insbesondere Braun- oder Schwarzglas, erhalten werden. Alternativ können an sich farblose Gläser durch einen sogenannten Beizprozess mit beispielsweise Silbernitrat-Lösungen, der temperaturgestützt sein kann, zumindest an den derart behandelten Oberflächen, eingefärbt werden. Derartige Gläser sind demnach nachträglich einfärbbar. Alternativ oder zusätzlich kann das Hüllmaterial auch eine lichtabsorbierende Beschichtung umfassen oder ein licht-absorbierend beschichtetes Glas umfassen oder daraus bestehen. Das Hüllmaterial des faseroptischen Lichtleiters umfasst also ein oder besteht aus einem farbigen Glas, bevorzugt Schwarz- oder Braunglas und/oder umfasst ein oder besteht aus einem einfärbbaren Glas, insbesondere schwarz oder braun einfärbbarem Glas, und/ oder das Hüllmaterial umfasst ein oder besteht aus einem licht-absorbierend beschichteten Glas.

Hier sei angemerkt, dass in Verbindung mit Kunststofflichtleitfasern (POF) das Hüllmaterial vorzugsweise eine licht-absorbierende Kunststoffhülse, ob eingefärbt oder farbig beschichtet, umfasst oder daraus besteht.

Es kann so also im Betriebszustand eines beispielsweise Endoskops mit erfindungsgemäßen Lichtleitern, Streulicht des in diese Lichtleiter eingekoppelten Lichtes, welches insbesondere seitlich aus dem Lichtleiter heraustritt, reduziert beziehungsweise minimiert und gegebenenfalls völlig verhindert werden. Die derartig licht-absorbierenden Materialien sind dabei gegebenenfalls an das anzuwendende Licht anzupassen, welches sowohl im sichtbaren (VIS) als auch im infraroten (IR) und/ oder ultravioletten (UV) Wellenlängenbereichs des Lichtspektrums liegen kann. So kann beispielsweise ein Übersprechen von Streulicht auf beziehungsweise in eine Kamera, die in ein Endoskop verbaut ist, verhindert oder zumindest reduziert werden und so die Darstellung des zu untersuchenden Objektes verbessern.

Besonders bevorzugt sind Lichtleitfasern aus einem bleifreien Glassystem für Kern und Mantel. Derartige Fasern werden sehr häufig im Bereich der Medizintechnik eingesetzt und sind unter anderem aus der US 11,034,612 B2 der Anmelderin bekannt. Je nach Variante der Faser, was sich insbesondere durch das Kernglas in Kombination mit dem optischen Mantel der Glasfaser beeinflussen lässt, können die Lichtleitfasern eine numerische Apertur NA von mind. 0,50, typisch im Bereich von 0,57, bevorzugt von mindestens 0,60, typisch im Bereich 0,64 bis 0,68 und besonders bevorzugt von mindestens 0,80, typisch 0,86, aufweisen, wobei eine höhere NA grundsätzlich für einen höheren übertragbaren Lichtstrom vorteilhaft ist. Zudem kann insbesondere bei endoskopischen Anwendungen eine bessere Ausleuchtung am distalen Ende des Lichtleiters erzielt werden.

Bei derartigen faseroptischen Lichtleitern sind beispielsweise, abhängig von den Durchmessern der einzelnen Lichtleitfasern, folgende Geometrien darstellbar: So kann ein typischer Durchmesser $d_1$ des Faserbündels im Bereich von 0,5 mm bis 3,0 mm, bevorzugt im Bereich von 2,5 mm bis 3,0 mm erzielt werden bei einem Durchmesser der Lichtleitfasern von $(30 \pm 4)\,\mu m$. Bei dickeren Lichtleitfasern, deren Durchmesser $(50 \pm 4)\,\mu m$ oder $(70 \pm 4)\,\mu m$ beträgt, kann der Durchmesser $d_1$ typischerweise im Bereich von 0,5 mm bis 8,0 mm, bevorzugt im Bereich von 2,5 mm bis 6,5 mm liegen. Dies sind Beispiele für typische Fasern. Grundsätzlich können auch davon abweichende Fasern mit größeren oder kleineren Durchmessern prozessiert und angewendet werden, wobei sich die bevorzugten Faserbündeldurchmesser dann zu eher größeren beziehungsweise kleineren Bereichen verschieben.

Die Dicke des Hüllmaterials liegt im bearbeiteten beziehungsweise verarbeiteten Zustand, also in dem davon umfassten und gegebenenfalls verjüngten Bereich des Lichtleiters im Bereich von 0,1 mm bis 0,5 mm, bevorzugt im Bereich von 0,15 mm bis 0,25 mm. Ein typischer Wert liegt bei etwa 0,2 mm, wobei bei dünneren Faserbündeln die Dicke tendenziell etwas dicker ist als bei größeren Faserbündeln.

Besonders vorteilhaft ist es, wenn die als Hüllmaterial ausgebildete Glashülse an der Seite, welche dem flexiblen Teil des Lichtleiters mit den Lichtleitfasern zugewandt ist, einen Kragen oder einen innen liegenden Konus aufweist, wodurch eine Einführhilfe für die Lichtleifasern beziehungsweise deren Bündel bereitgestellt wird. Diese bevorzugte Ausgestaltung hat den Vorteil, dass die Lichtleitfasern mit wenig Aufwand dicht gepackt in die Glashülse montiert, also eingeführt werden können. Es genügt bereits, wenn diese Einführhilfe nur wenige zehntel Millimeter als solche ausgeprägt ist.

In einer weiteren bevorzugten Ausgestaltung, kann vorgesehen sein, dass zumindest das verformte und/ oder verjüngte Ende des faseroptischen Lichtleiters mit der verjüngten Endfläche am Verjüngungsbereich und das andere Ende mit der ursprünglichen Endfläche, die sich an den Bereich mit dem im Wesentlichen konstanten Durchmesser $d_1$ anschließt, in jeweils einer Endhülse aus Metall oder Kunststoff montiert sind und sich zwischen dem faseroptischen Lichtleiter und der Innenseite der Endhülsen zumindest bereichsweise eine Schicht aus Kunststoff befindet. Dieser Kunststoff ist üblicherweise als Vergussmasse oder als Kleber ausgebildet. Die jeweiligen Endhülsen stellen neben einer mechanischen Schutzfunktion für die Enden des Lichtleiters eine mechanische Schnittstelle zum Anschluss an eine Lichtquelle und/ oder an ein Instrument dar, und können beispielsweise Schraubgewinde, Bajonette-Anschlüsse oder Steckbeziehungsweise Rastverbindungen aufweisen.

Eine bevorzugte Verwendung des faseroptischen Lichtleiters, wie zuvor beschrieben ist, sieht den Einsatz in einem medizintechnischen oder industriellen Endoskop-Instrument und/ oder als Lichtleiter zwischen einer Lichtquelle und dem Endoskop-Instrument oder einer Analyse-Einrichtung oder als Lichtleiter zwischen einem Behälter und einer Detektor-Einheit vor. Insbesondere können in Verbindung mit Lichtquellen, welche Halogen-, LED- und/ oder Laser-basierte Leuchtmittel enthalten können, aufgrund der besonders guten optischen Übertragungseigenschaften der Glasfasern der Anmelderin sehr hohe Leuchtstärken mit geringem Farb-Shift, also Verfälschung der farblichen Übertragung, erzielt werden. Dies kann beispielsweise auch als Unterschied von Farbtemperaturen angegeben werden. Zudem können Beleuchtungssysteme mit mehreren hintereinander geschalteten lichtleitenden Komponenten, wie dies insbesondere bei Endoskopen, ob wieder verwendbar/ wieder aufbereitbar oder als Single-Use-Endoskop ausgelegt, der Fall ist, Vorteile bei der gesamten Lichtübertragungseffizienz erzielt werden. Weitere Anwendungsbereiche können im Dental-medizinischem Umfeld, im chirurgischen Umfeld oder im dermatologischen Umfeld in der Medizintechnik, insbesondere auch im Bereich roboterassistierter medizinischer Untersuchungsmethoden oder Operationsmethoden, auch "Robotic Surgery" genannt, liegen. Dabei kann die gesamte Länge des faseroptischen Lichtleiters typischerweise bis zu 6 m, in manchen Fällen bis zu 10 m und mehr betragen. Vorteilhaft sind dann insbesondere die zuvor beschriebenen Glasfaser-basierten Lichtleiter, die eine geringe Farbverschiebung bei derartigen Anwendungslängen aufweisen.

Gegenstand der Erfindung ist auch ein Verfahren gemäß Anspruch 12.

Die das Verfahren betreffende Aufgabe wird dadurch gelöst, dass zum Herstellen eines faseroptischen Lichtleiters, welcher eine Mehrzahl von Lichtleitfasern aufweist, welche als flexible Faserbündel mit im Wesentlichen konstantem Durchmesser $d_1$ zusammengefasst sind, die nachfolgenden Verfahrensschritte umfasst sind:

- Einbringen der Lichtleitfasern als Faserbündel in ein Hüllrohr, welches das Faserbündel auf einer Teillänge umschließt,
- Erwärmen des in dem Hüllrohr befindlichen Faserbündels und des Hüllrohres auf zumindest einer Teillänge des Hüllrohres bis mindestens zum Erweichen der Lichtleitfasern und des Hüllrohres,
- kollabieren des Hüllrohres auf das Faserbündel der Lichtleitfasern, wobei das Kollabieren durch Pressen und/ oder beispielsweise Unterdruck unterstützt werden kann, so dass ein starrer Bereich ausgebildet wird, sowie
- Verformen des Lichtleiters im starren Bereich aus seiner Längsachse heraus beziehungsweise aus der Richtung der ursprünglichen Längsachse heraus und/ oder Umformen seines Querschnitts beziehungsweise seiner Querschnittsgeometrie.

Mit diesen Verfahrensschritten lassen sich unterschiedliche Geometrien hinsichtlich einer axialen Verformung des Lichtleiters als auch hinsichtlich einer Querschnittsgeometrieänderung entlang der Achse des Lichtleiters realisieren, wobei hier die Querschnittsfläche konstant ist.

Mit anderen Worten wird so ein Faseroptischer Lichtleiter, beinhaltend eine Mehrzahl von Lichtleitfasern, welche als flexible Faserbündel mit im Wesentlichen konstantem Durchmesser $d_1$ zusammengefasst beziehungsweise zusammenfassbar sind und an mindestens einem Ende des faseroptischen Lichtleiters zumindest in Teilbereichen seiner Länge vollumfänglich von einem Hüllmaterial umschlossen sind, wodurch ein starrer Abschnitt mit einer Längsachse (16) gebildet ist, erhalten. Dabei sind die Lichtleitfasern in dem starren Abschnitt miteinander und vorzugsweise auch mit dem Hüllmaterial verschmolzen, und der faseroptische Lichtleiter ist im Bereich des Hüllmaterials zumindest einmal aus seiner Längsachse heraus gebogen und/ oder die Querschnittsgeometrie des faseroptischen Lichtleiter ist im Bereich des Hüllmaterials zumindest einmal umgeformt.

Hinsichtlich einer zusätzlichen Querschnittsflächenänderung sieht eine ebenfalls bevorzugte Verfahrensvariante vor, dass nach dem Erwärmen des in dem Hüllrohr befindlichen Faserbündels und des Hüllrohres zusätzlich folgende Verfahrensschritte durchgeführt werden:

- Ausziehen des erwärmten Bereichs, bis das in dem Hüllrohr befindliche Faserbündel, insbesondere zusammen mit dem Hüllmaterial (20), in zumindest einem Teilbereich den Durchmesser $d_2$ aufweist und so ein Verjüngungsbereich gebildet wird, in dem die Faserstruktur nicht unterbrochen wird,
- Abtrennen des Hüllrohres und des darin befindlichen Faserbündels im Verjüngungsbereich an der oder einer Stelle des Durchmessers $d_2$.

Daraus ergibt sich zumindest ein Lichtleiter mit einem von einem Hüllmaterial umschlossenen, damit starren Bereich, welcher einen Bereich mit einem Durchmesser $d_1$ und einen Verjüngungsbereich von $d_1$ auf einen Durchmesser $d_2$ aufweist, wobei der Verjüngungsbereich beziehungsweise verjüngte Bereich eine Länge $l_2$ aufweist.

Daran schließt sich der zuvor beschriebene Verform-Prozess, bezüglich zumindest einer Biegung und/ oder zumindest einer Umformung beziehungsweise Änderung der Querschnittsgeometrie an. Damit kann auch ein an seinem Ende geformter Lichtleiter realisiert werden, dessen Querschnittsfläche zum Ende hin verkleinert ist. Die Verformung findet dabei in weiter bevorzugten Ausführungsformen im Bereich mit der Länge $l_2$ statt.

Es liegt so ein Faseroptischer Lichtleiter vor, wobei der faseroptische Lichtleiter einen flexiblen Bereich der Länge $l_1$ mit im Wesentlichen konstantem Durchmesser $d_1$ an seiner ursprünglichen Endfläche aufweist und zumindest einen sich daran anschließenden starren Verjüngungsbereich der Länge $l_2$, in dem sich der Durchmesser des Lichtleiters von dem Durchmesser $d_1$ auf den Durchmesser $d_2$ verringert. Dabei ist der Verjüngungsbereich zumindest in Teilbereichen seiner Länge vollumfänglich von dem Hüllmaterial umschlossen und die Faserstruktur setzt sich ohne Unterbrechung von dem Bereich mit dem im Wesentlichen konstanten Durchmesser in den Verjüngungsbereich fort, wobei der Verjüngungsbereich eine verjüngte Endfläche aufweist, in der die Lichtleitfasern miteinander und vorzugsweise auch mit dem Hüllmaterial verschmolzen sind, und wobei der faseroptische Lichtleiter im Bereich des Hüllmaterials zumindest einmal aus seiner Längsachse heraus gebogen und/ oder die Querschnittsgeometrie des faseroptischen Lichtleiter im Bereich des Hüllmaterials zumindest einmal umgeformt ist.

In weiteren Ausführungsformen kann bei den zuvor beschriebenen Verfahrensschritten vorgesehen sein, dass das eingesetzte Hüllrohr bereits einen unrunden, insbesondere D-förmigen, Querschnitt aufweist, und so bereits als solches als unrundes Rohr vorliegt oder das Rohr beispielsweise in einem vorgeschalteten Prozess aus seinem an sich runden Querschnitt heraus in seinem Querschnitt über die gesamte Länge unrund, beispielsweise oval oder im wesentlichem D-förmig verformt wurde. Ein derart geformtes beziehungsweise umgeformtes Hüllrohr hat den Vorteil, dass ein geringerer Umformgrad der lichtleitenden Fasern beim Ziehen, Pressen, Verjüngen, Biegen oder Umformen des Lichtleiters erzielt werden kann, was vorteilhaft hinsichtlich einer Reduzierung der Lichtverluste ist. Zudem ergeben sich Vorteile hinsichtlich einer deutlich dichteren Verschmelzung und beim Verpressen. Ein weiterer Vorteil ergibt sich gegenüber einem runden Hüllglas mit nur am Ende verformten Abschnitten, dass ein über die gesamte Länge vorgeformtes Hüllrohr bzw. ein darauf basierenden Lichtleiter günstiger im oft nur spärlich zur Verfügung stehenden Bauraum im Endoskop verbaut werden kann.

In einer weiteren Verfahrensvariante kann zusätzlich vorgesehen sein, dass das Hüllrohr einseitig verschlossen wird und vor dem Verformen oder dem Ausziehen des erwärmten Bereichs Unterdruck an das Hüllrohr und die Lichtleitfasern angelegt wird, so dass die Lichtleitfasern miteinander, vorzugsweise auch mit dem Hüllrohr, verschmelzen. Dies dient der zusätzlichen Komprimierung der Lichtleitfasern, so dass diese nahezu ideal anliegen und Luftzwischenräume, sogenannte Zwickel weitgehend vermieden werden, was das Eindringen von Körperflüssigkeiten insbesondere bei medizinischen Endoskopen beispielsweise oder das Eindringen von Reinigungsflüssigkeiten beispielsweise bei der Aufbereitung verhindert.

Wird dabei der erwärmte Bereich vor dem Abtrennen des Hüllrohres und des darin befindlichen Faserbündels abgekühlt, kann der Lichtleiter zum Beispiel an seinen Endflächen oder die den abgetrennten Verjüngungsbereich abschließende verjüngte Endfläche geschliffen und/ oder poliert werden. Damit kann eine hochwertige optische Qualität der Faserendflächen erzielt werden.

Die Erfindung wird im Folgenden anhand eines in den Figuren dargestellten Ausführungsbeispiels näher erläutert. Es zeigen:

Fig. 1 schematisch einen verjüngten faseroptischen Lichtleiter,
Fig. 2 eine Mikroskopaufnahme der verjüngten Endfläche des Lichtleiters,
Fig. 3a bis 3i schematisch Beispiele für einen 3D-verformten Bereich des Lichtleiters,
Fig. 4a bis 4f schematisch als Abfolge den Herstellprozess Lichtleiters mit einer runden verjüngten Endfläche des Lichtleiters und

Fig. 5 schematisch den Umformprozess am starren Ende des faseroptischen Lichtleiters

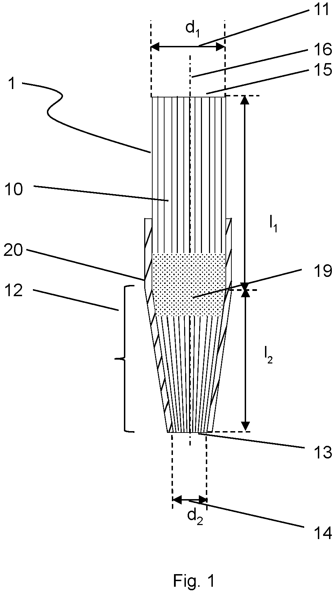

Figur 1 zeigt schematisch den Längsschnitt durch einen faseroptischen Lichtleiter 1. Dieser besteht aus den Lichtleitfasern 10, welche sich ohne Unterbrechung der Faserstruktur entlang der Längsachse 16 von dem Bereich mit dem im Wesentlichen konstanten Durchmesser $d_1$ 11 und der Länge $l_1$ in den Verjüngungsbereich 12 fortsetzen, welcher die Länge $l_2$ aufweist und in dem sich der Durchmesser des faseroptischen Lichtleiters 1 von $d_1$ 11 auf einen Durchmesser $d_2$ 14 verringert. Der Verjüngungsbereich 12 wird durch eine verjüngte Endfläche 13 abgeschlossen, der Bereich mit dem im Wesentlichen konstanten Durchmesser $d_1$ 11 durch eine ursprüngliche Endfläche 15.

Entlang seiner Außenumfangsfläche ist der Verjüngungsbereich 12 vollständig von einem Hüllmaterial 20 umgeben. Entsprechend der dargestellten Ausführungsform erstreckt sich das Hüllmaterial 20 auch über den Verjüngungsbereich 12 in den Bereich mit dem im Wesentlichen konstanten Durchmesser $d_1$ 11 hinein. Wie bereits beschrieben weist das Hüllmaterial 20 bevorzugt einen thermischen Ausdehnungskoeffizienten auf, der dem des Materials der Lichtleitfaser 10 angepasst ist. Wie ebenfalls bereits beschrieben können die Lichtleitfasern 10 ungeordnet sein, was bedeutet, dass die relative Position der einzelnen Lichtleitfasern in der Eingangsmatrix der Lichtleitfasern an der ursprünglichen Endfläche 15 nicht mit der relativen Position der Lichtleitfasern in der Ausgangsmatrix an der verjüngten Endfläche 13 übereinstimmen. Diese ungeordnete Anordnung kann bei Beleuchtungsanwendungen wie in der Endoskopie sogar beabsichtigt sein und gezielte Unordnungen erzeugt und eingesetzt werden, weil durch die Unordnung der einzelnen Lichtleitfasern 10 besonders homogene Helligkeitsverteilungen an der als Lichtaustrittsfläche ausgebildeten verjüngten Endfläche 13 erreicht werden können. Wird Licht in die ursprüngliche Endfläche 15 eingekoppelt und an der verjüngten Endfläche 13 des Verjüngungsbereichs 12 ausgekoppelt, wirkt der faseroptische Lichtleiter 1 durch die Querschnittswandlung quasi als Lichtkonzentrator. Selbstverständlich ist es ebenso möglich, das Licht an der verjüngten Endfläche 13 ein- und an der ursprünglichen Endfläche 15 auszukoppeln. Als Längsachse 16 wird hierbei die Achse angesehen, die durch den mit dem Hüllmaterial 20 umgebenen, somit starren, Bereich vorgegeben wird beziehungsweise vorgebbar ist, wie dies auch den Figuren 1, 3a-i, 4a und 5.a entnehmbar ist. Der flexible Bereich ist vereinfachend der Längsachse 16 folgend dargestellt.

Ebenso ist es erfindungsgemäß möglich, dass für den erfindungsgemäßen faseroptischen Lichtleiter 1 die Lichtleitfasern 10 so angeordnet werden, dass die relative Lage einer einzelnen Lichtleitfaser 10 in der Eingangsmatrix der ursprünglichen Endfläche 15 der relativen Lage der Lichtleitfaser 10 in der Ausgangsmatrix der verjüngten Endfläche 13 entspricht. In diesem Fall spricht man von geordneten Faserbündeln. Diese sind in der Lage, Bildinformationen zu übertragen. Wird Bildinformation an der größeren ursprünglichen Endfläche 15 eingekoppelt, kann das Bild an der kleineren verjüngten Endfläche 13 verkleinert empfangen werden. In umgekehrter Einkopplungsrichtung wird eine Vergrößerung des Bildes erreicht.

Wie ebenfalls bereits beschrieben können die Lichtleiter 10 starr oder flexibel sein. Auch hybride Lösungen, in denen Teilbereiche starr und Teilbereiche flexibel sind, sind möglich. Der mit dem Hüllmaterial 20 umgebene Bereich des faseroptischen Lichtleiters 1 ist in der Regel starr, wobei sich im Bereich des Hüllmaterials 20 ein Übergangsbereich 19 ausbildet, in dem der flexible Bereich der Lichtleitfasern 10 in den starren, verschmolzenen Bereich übergeht.

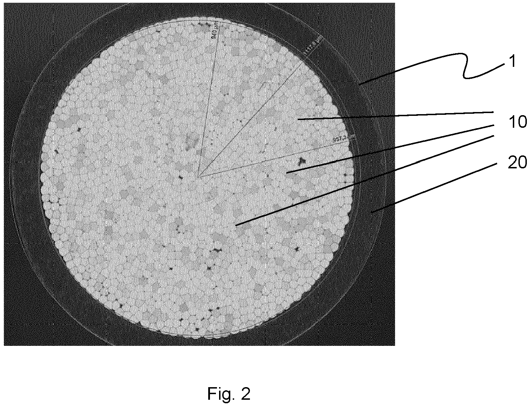

Figur 2 stellt eine fotografische Aufnahme der verjüngten Endfläche 13 am Ende des Verjüngungsbereichs 12 des erfindungsgemäßen faseroptischen Lichtleiters 1 dar. Wie zu erkennen ist, sind die Lichtleitfasern 10 an ihrer gemeinsamen Außenumfangsfläche von dem Hüllmaterial 20 ringförmig umschlossen und das Hüllmaterial 20 trägt so zur Stabilisierung der Lichtleitfasern 10 bei. Die Lichtleitfasern 10 sind hier miteinander verschmolzen. Dies wurde durch das Ausziehen des Verjüngungsbereichs 12 unter dem Anliegen von Unterdruck erreicht. Durch den Unterdruck werden die Lichtleitfasern aneinandergepresst und nehmen die ersichtliche im Wesentlichen polygonale, insbesondere im Wesentlichen hexagonale Form an. Zwischen den verschmolzenen Lichtleitfasern 10 befinden sich bevorzugt keine Zwischenräume mehr, so dass kein Medium mehr in die Endfläche 13 eindringen kann. Dies hat insbesondere Vorteile bei medizinischen Anwendungen des erfindungsgemäßen Lichtleiters 1, da dieser so besser autoklavierbar wird.

Am anderen Ende des Lichtleiters 1 beziehungsweise in dem Teil des Lichtleiters 1, der nicht von einem Hüllmaterial umschlossen ausgebildet ist oder wird, ist in der Regel vorgesehen, dass die Lichtleitfasern 10 nicht notwendigerweise miteinander verschmolzen sind und daher als flexibles Faserbündel anzusehen sind. Die Lichtleitfasern 10 weisen nach wie vor einen runden Querschnitt auf und es befinden sich Zwischenräume zwischen diesen.

Üblicherweise ist der Lichtleiter 1 an seinen Enden in entsprechende Endhülse eingefasst beziehungsweise montiert, um den Lichtleiter 1 an andere Objekte wie beispielsweise weitere Lichtleiter und/ oder Lichtquellen und/ oder Messgeräte beziehungsweise Endoskop-Instrumente usw. anzuschließen oder anschließbar zu machen. Diese Endhülsen, welche aus Metall, zum Beispiel aus Edelstahl, oder Kunststoff, zum Beispiel aus PPS, PPSU, PC, bestehen können, sind im Inneren derart ausgeformt, dass sie zum Beispiel den verjüngten Bereich des Lichtleiters aufnehmen. Zur Fixierung dienen in der Regel Vergussmassen, beispielsweise 1- oder 2-komponentige Kleber.

In weiteren Ausführungsformen ist vorteilhaft vorgesehen, dass zumindest der flexible Teil des Lichtleiters 1 beziehungsweise die darin befindlichen Lichtleitfasern 10 von einer flexiblen Umhüllung, beispielsweise einem Schlauch, Gewebe oder Geflecht aus Polymeren oder Metallen oder einer Kombination daraus, beispielsweise als Spiralschlauch umfasst werden. Die derartige flexible Umhüllung kann auch an die Endhülsen und/oder den starren, mit einem Hüllmaterial 20 umgebenen Teil des Lichtleiters 1 anschließen und/oder diese je zumindest teilweise umfassen. Diese flexible Umhüllung kann auch sogenanntes Schrumpfschlauchmaterial umfassen oder daraus bestehen und/ oder auch mehrteilig aus unterschiedlichen Materialien, die sich auch zumindest teilweise überlappen können, ausgebildet werden.

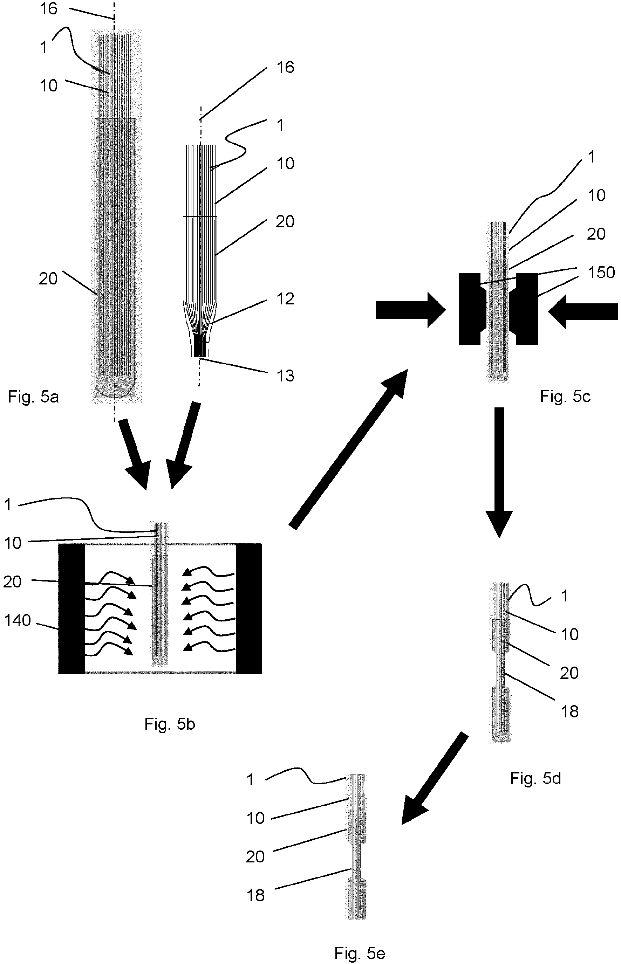

Gemäß der Erfindung kann weiterhin vorgesehen sein, dass der faseroptische Lichtleiter an seinem Ende nicht verjüngt wird, sondern dass das als Glashülse ausgebildete Hüllmaterial 20 für eine weitere Verformung des einen Endes des Lichtleiters 1 die Lichtleitfasern 10 umschließt und während des Herstellprozesses auf das Bündel der Lichtleitfasern 10 kollabiert und die Lichtleitfasern 10 derart verpresst, dass diese, wie in Fig. 2 gezeigt, eine im Wesentlichen dichte hexagonale Packung aufweisen. Danach kann durch einen weiteren Verformungsschritt mittels eines Presswerkzeuges 150 (siehe Figur 5) eine von der Zylinderform abweichende Kontur (Umformung der Querschnittsgeometrie) und/ oder eine gezielte Biegung des bis dahin zylinderförmigen Abschnittes des Lichtleiters 1 aus seiner ursprünglichen Längsachse 16 heraus erreicht werden.

Hier sei angemerkt, dass dieser Verformungsprozess auch an zuvor kegelförmig verjüngten Lichtleitern 1 angewendet werden kann und man damit einen verjüngten und gleichzeitig verformten faseroptischen Lichtleiter 1 erhält.

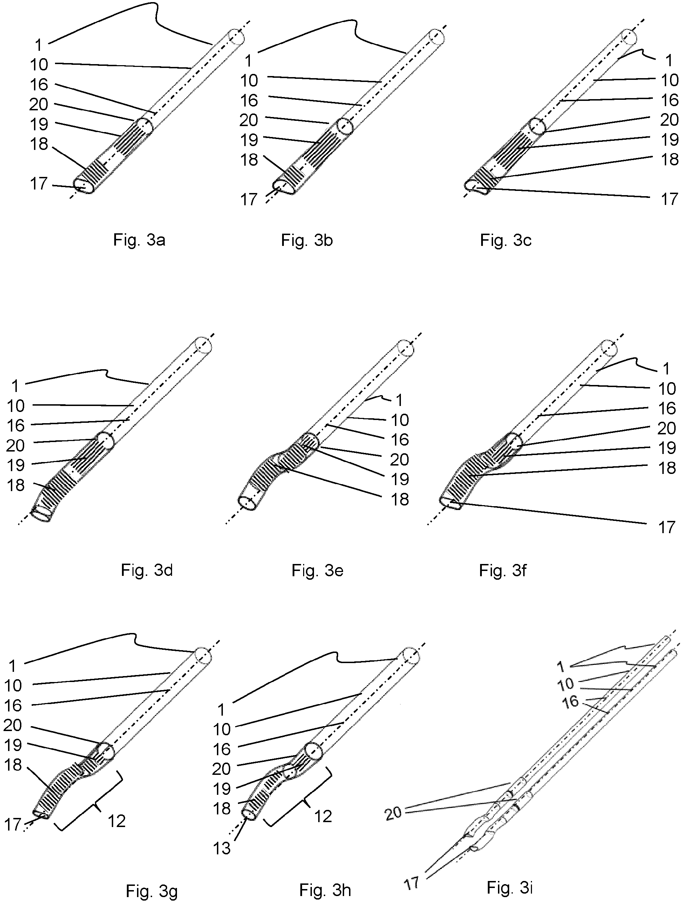

Die Figuren 3a bis 3i zeigen entsprechende Ausführungsformen eines derart hergestellten Lichtleiters 1. Dargestellt sind jeweils faseroptische Lichtleiter 1, welche aus einem Bündel aus Lichtleitfasern 10 bestehen und an deren einem Ende der Lichtleiter 1 einen Verformungsbereich 18 aufweist, wobei der Lichtleiter 1 am Ende einen Bereich aufweist, in dem dieser vollumfänglich von dem als Hüllrohr ausgebildeten Hüllmaterial 20 umschlossen ist und die Lichtleitfasern 10 im Bereich des Hüllmaterials 20 miteinander und vorzugsweise auch mit dem Hüllmaterial 20 verschmolzen sind und einen starren Abschnitt bilden. Dabei bildet sich ein Übergangsbereich 19 aus, bei denen die Lichtleitfasern 10 von einem unverschmolzenen Bereich, in dem das Faserbündel flexibel beziehungsweise biegsam ist, in den verschmolzenen, das heißt starren Bereich zum Ende des Lichtleiters 1 übergehen. Dieser Übergangsbereich 19 als auch der Verformungsbereich 18 sind vollständig vom Hüllmaterial 20 umschlossen und dies sorgt für eine ausreichende mechanische Stabilisierung des Übergangbereichs 19. Mittels eines Umformprozesses, wie in den Figuren 5a bis 5e beschrieben ist, kann erfindungsgemäß mittels Einwirkung von beispielsweise Druck und Temperatur der Lichtleiter 1 aus seiner Längsachse 16 heraus gebogen und/ oder hinsichtlich seiner Querschnittgeometrie umgeformt werden.

Die Fig. 3a bis 3c zeigen Beispiele, bei denen der Lichtleiter 1 bei konstanter Querschnittsfläche entlang seiner Längsachse 16 am Ende verformt ist und eine von der hier Kreisform abweichende verformte bzw. umgeformte Endfläche 17 aufweist. Beispielhaft ist in Fig. 3a eine verformte bzw. umgeformte Endfläche 17 mit ovaler Geometrie, oder auch flacher Verpressung, gezeigt. Fig. 3b zeigt eine verformte bzw. umgeformte Endfläche 17 mit im Wesentlichen D-förmiger Geometrie und Fig. 3c eine verformte bzw. umgeformte Endfläche mit nierenförmiger Geometrie. Weiterhin denkbar sind im Wesentlichen polygonale, insbesondere quadratische, rechteckige, trapezförmige sowie dreieckige Geometrien. Grundsätzlich lassen sich beliebige Geometrien darstellen, wobei es allerdings je nach Material der Lichtleitfasern 10 des Lichtleiters 1 aufgrund der temperaturabhängigen Viskositätseigenschaften des Materials beim Verformungsprozess Grenzen hinsichtlich der Ausbildung von scharf ausgebildeten Kanten beziehungsweise Ecken oder zum Beispiel spitz zulaufenden Geometrien gibt. Die sich darstellenden Geometrien weisen daher immer leichte Verrundungen in derartigen Bereichen auf und entsprechen so im Wesentlichen den zu erzielenden, geforderten oder erwünschten Geometrien.

Weiterhin kann vorgesehen sein, dass der Lichtleiter 1 bei konstanter Querschnittsfläche entlang seiner ursprünglichen Längsachse 16 aus dieser Längsachse 16 heraus verformt ist. Mit anderen Worten ist der Lichtleiter 1 damit zumindest ein-fach gebogen oder abgewinkelt ausgebildet. Fig. 3d zeigt ein Beispiel, bei dem der Lichtleiter am Ende abgewinkelt wurde, das heißt eine Biegung erfahren hat. Fig. 3e zeigt ein Beispiel, bei dem der Lichtleiter zwei Biegebereiche aufweist.

In Fig. 3f ist ein Lichtleiter 1 beispielhaft dargestellt, der zum einen 2 Biegungen bezogen auf seine Längsachse 16 aufweist und zum anderen an seinem Ende zusätzlich eine verformte Endfläche 17, hier mit ovaler Geometrie gezeigt, aufweist.

Die Fig. 3g und 3h zeigen Beispiele für einen verformten Lichtleiter 1 bei dem zusätzlich der Querschnitt des Lichtleiters zu seinem verformten Ende hin in einem Verjüngungsbereich 12 verjüngt ist. Neben zwei Biegungen aus der Längsachse 16 heraus weist der in Fig. 3g gezeigte Lichtleiter 1 zudem eine oval verformte bzw. umgeformte Endfläche 17 auf. Der in Figur 3h gezeigte Lichtleiter weist hingegen lediglich eine verjüngte Endfläche 13 mit noch kreisrundem Querschnitt auf.

In Fig. 3i sind beispielhaft zwei Lichtleiter 1 dargestellt, die jeweils ähnlich wie der in Fig. 3g gezeigte Lichtleiter 1 verformt ist, das heißt eine Kombination aus Biegungen und verformten Endflächen 17 aufweisen. Die Anordnung der beiden Lichtleiter 1 ist dabei derart gewählt, dass zwischen den Lichtleitern, beispielsweise in einem Endoskop-Instrument verbaut, Arbeitskanäle und/ oder elektrische Zuleitungen zu einem Kamera-Chip oder einem faseroptischen Bildleiter, zwischen den verformten Endflächen 17 erfindungsgemäßen Lichtleiter angeordnet sein können (in Fig. 3i nicht dargestellt). Mit der gezeigten Ausgestaltung am jeweiligen Ende des jeweiligen Lichtleiters 1 kann erreicht werden, dass das zu untersuchende Objekt beispielsweise eine Gewebeoberfläche schattenfrei ausgeleuchtet werden kann.

Es sei darauf hingewiesen, dass die in den Fig. 3a bis 3i gezeigten Ausführungen lediglich Beispiele darstellen. Es können beliebige Kombination aus Querschnittsänderung und Verformung des Lichtleiters 1 erzielt werden. So läßt sich ein an sich kreisrunder Querschnitt des Lichtleiters 1 auch nur in einem bestimmten Bereich z.B. flach pressen, wie dies schematisch beim Umformverfahren in den Fig. 5a bis 5e gezeigt ist.

Das Verfahren zum Herstellen eines faseroptischen Lichtleiters 1, wie zuvor beschrieben wurde, der eine Mehrzahl von Lichtleitfasern 10 aufweist, welche als flexible Faserbündel mit im Wesentlichen konstantem Durchmesser $d_1$ 11 zusammengefasst sind, umfasst die folgenden Haupt-Verfahrensschritte:

- Einbringen der Lichtleitfasern 10 als Faserbündel in ein Hüllrohr 21, welches das Faserbündel auf einer Teillänge umschließt,
- Erwärmen des in dem Hüllrohr 21 befindlichen Faserbündels und des Hüllrohres 21 auf zumindest einer Teillänge des Hüllrohres bis mindestens zum Erweichen der Lichtleitfasern 10 und des Hüllrohres 21,
- kollabieren des Hüllrohres 21 auf das Faserbündel der Lichtleitfasern 10 durch beispielsweise Unterdruck und/ oder einwirken einer Kraft, sodass sich ein starrer Bereich ausbildet, in dem das Hüllrohr 21 und die Lichtleitfaser 10 mit dem Hüllrohr 21 und sich selbst zumindest teilweise verschmolzen sind, und das Hüllrohr 21 das Hüllmaterial 20 bildet und
- Verformen beziehungsweise Biegen des Lichtleiters 1 im Bereich des Hüllrohres 21 aus seiner Längsachse 16 heraus und/ oder Umformen hinsichtlich seiner Querschnittsgeometrie.

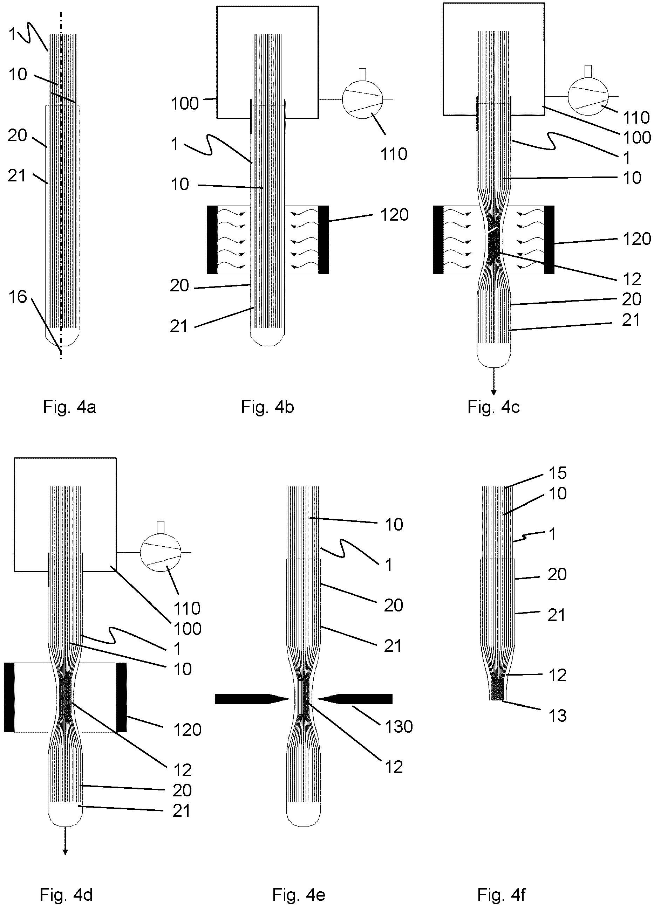

Dies gilt, wenn die Querschnittsfläche entlang der Längsachse 16 des Lichtleiters 1 konstant bleibt und ist schematisch in den Fig. 4a und b dargestellt, an die sich dann die in Figur 5a bis 5e ebenfalls schematisch gezeigten Umform-Prozessschritte anschließen.

Soll zusätzlich eine Verjüngung vorgesehen werden, bei der sich die Querschnittsfläche zum Ende 13 hin verkleinert, werden nach dem Erwärmen der in dem Hüllrohr 21 befindlichen Faserbündels und des Hüllrohres 21 zusätzlich folgende Verfahrensschritte vor dem Verformungsprozess durchgeführt:

- Ausziehen des erwärmten Bereichs, bis das in dem Hüllrohr 21 befindliche Faserbündel bzw. das so vom Hüllmaterial 20 umschlossene Faserbündel, also zusammen mit dem Hüllmaterial (20), in zumindest einem Teilbereich den Durchmesser $d_2$ 14 aufweist und so ein Verjüngungsbereich 12 gebildet wird, in dem die Faserstruktur nicht unterbrochen wird,
- Abtrennen des Hüllrohres 21 und des darin befindlichen Faserbündels im Verjüngungsbereich (12) an der Stelle des Durchmessers $d_2$.

Die Figur 4a bis 4f zeigt damit schematisch die Abfolge der Verfahrensschritte für einen Lichtleiter 1 inklusive einer Verjüngung der Querschnittsfläche, an die sich dann die in Figur 5a bis 5e ebenfalls schematisch gezeigten Umform-Prozessschritte anschließen.

Figur 4a stellt den ersten Verfahrensschritt dar, nach dem die Lichtleitfasern 10 in das Hüllmaterial 20, welches als Hüllrohr 21 ausgebildet ist, eingebracht werden und so ein Faserbündel bilden. In der Figur 4a wird ein einseitig geschlossenes Hüllrohr 21 verwendet, welches zuvor durch bekannte Methoden hergestellt wurde. Da das Hüllrohr 21 als das den Verjüngungsbereich 12 umgebende Hüllmaterial 20 am erfindungsgemäßen Lichtleiter 1 verbleibt, wird sein Material bevorzugt so ausgewählt, dass sein thermischer Ausdehnungskoeffizient dem der Lichtleitfasern 10 angepasst ist. Werden Kern-Mantelfasern verwendet, wird der thermische Ausdehnungskoeffizient des Kernmaterials zur Anpassung herangezogen, da er üblicherweise den Hauptbestandteil der einzelnen Kern-Mantel Lichtleitfaser 10 ausmacht. Die Lichtleitfasern 10 selbst können zuvor in bekannten Ziehprozessen hergestellt und auf die benötigte Länge abgelängt werden. Dadurch können auch Standard-Lichtleitfasern für den erfindungsgemäßen faseroptischen Lichtleiter 1 eingesetzt werden.

Figur 4b stellt einen weiteren Verfahrensschritt dar. Die in dem Hüllrohr 21 eingebrachten Lichtleitfasern 10 werden dabei an ihren aus dem Hüllrohr 21 herausstehenden Ende von einer Unterdruckkammer 100 umgeben, die so ausgelegt ist, dass sie dem anzulegenden Unterdruck zu widerstehen vermag. Dies kann beispielsweise ein Metallrohr sein. Die Verbindung zwischen Hüllrohr 21 und Unterdruckkammer 100 wird mit geeigneten Maßnahmen abgedichtet, so dass beim Abpumpen der Luft aus dem durch die Unterdruckkammer 100 und dem Hüllrohr 21 gebildeten Volumen mittels der Pumpe 110 keine oder zumindest nicht störend viel Luft durch die Verbindungsstelle dringen kann. Zumindest eine Heizeinrichtung 120 ist so angebracht, dass es einen Teilbereich des Hüllrohres 21 und der darin eingebrachten Lichtleitfasern 10 erwärmen kann. Als Heizeinrichtung 120 kann beispielsweise ein elektrischer Ringofen verwendet werden, aber auch andere Vorrichtungen wie Brenner oder Laserstrahlung und Ähnliches sind dem Fachmann bekannt und können eingesetzt werden. Die Heizeinrichtung 120 und/ oder die Vorrichtung, an der das Hüllrohr 21 mit den Lichtleitfasern 10 angebracht oder anbringbar sind, können verfahrbar ausgelegt sein, so dass das Kollabieren und/ oder Verschmelzen des Hüllrohres auf oder mit den Lichtleitfasern 10 auch über eine gewünschte Länge oder an bestimmten Positionen erfolgen kann. Das verschlossene Ende des Hüllrohres wird in der Regel abgetrennt (nicht dargestellt) und es wird so ein faseroptischer Lichtleiter 1 mit zunächst im Wesentlichen konstantem Durchmesser $d_1$ 15 erhalten.

Ein Lichtleiter 1 mit einem Verjüngungsbereich 12 mit einem Durchmesser $d_2$ 14 wird durch Anwendung weiterer Verfahrensschritte erhalten. In Figur 4c ist das Ausziehen des Verjüngungsbereichs 12 als weiterer Verfahrensschritt dargestellt. Sind das Hüllrohr 21 und die darin befindlichen Lichtleitfasern 10 ausreichend erwärmt, wird das Hüllrohr 21 durch geeignete ebenfalls bekannte Maßnahmen entlang der Achse der Lichtleitfasern 10 ausgezogen. Durch die dadurch bedingte Verlängerung des erwärmten Bereichs schnürt sich dieser ein und es wird der Verjüngungsbereich 12 gebildet. Je länger ausgezogen wird, desto kleiner wird der Durchmesser des ausgezogenen Bereichs. Durch die Länge des Auszugs kann der gewünschte minimale Durchmesser $d_2$ 14 eingestellt werden. Liegt wie zuvor beschrieben im Innern der Unterdruckkammer 100 und des Hüllrohrs 21 Unterdruck an, wirkt der Druck des umgebenden Mediums, in der Regel der Luftdruck, eine Kraft senkrecht zur Faserachse der Lichtleitfasern 10 auf diese aus und bewirkt so, dass die Lichtleitfasern 10 untereinander und mit der inneren Wandung des Hüllrohres 21 unter Druck verschmelzen.

Durch geeignete Führung dieses Prozesses, beispielsweise mittels hinsichtlich deren Temperaturprofil variabler Heizeinrichtungen 120 mit gegebenenfalls mehreren Heizzonen oder einer oder mehreren verfahrbaren Heizeinrichtungen, kann ein Verjüngungsbereich 12 erreicht werden, der nach der eigentlichen Verjüngung auch einen im Wesentlichen gleichbleibenden Durchmesser d2 aufweist.

In einem weiteren Verfahrensschritt nach Figur 4d wird der zuvor erwärmte Bereich abgekühlt. Dies kann je nach Materialwahl der Lichtleitfasern 10 und des Hüllrohres 21 bevorzugt kontrolliert erfolgen, das heißt, dass nicht einfach die Heizeinrichtung 120 abgeschaltet wird, sondern dass langsam ihre Heizleistung reduziert wird, um ein Platzen oder Reißen des zuvor erwärmten Bereichs zu verhindern. Außerdem ist es möglich, während des Abkühlens weiterhin eine Kraft in Richtung der Auszugsrichtung auf den faseroptischen Lichtleiter 1 auszuüben, damit sich dieser während des Abkühlvorgangs nicht über Gebühr kontrahiert und sich so der Durchmesser $d_2$ 14 gegebenenfalls unerwünscht verändern würde.

In einem weiteren Verfahrensschritt nach Figur 4e werden die Heizeinrichtung 120 und die Unterdruckkammer 100 entfernt und der ausgezogene Bereich wird an der einer passenden Stelle mit einer geeigneten Trenneinrichtung 130 abgetrennt. Dazu wird die Stelle gewählt, an der der ausgezogene Bereich den gewünschten Durchmesser $d_2$ 14 aufweist, ggfls. zuzüglich für die Nachbearbeitung benötigter Toleranzen.

Das Ergebnis des erfindungsgemäßen Herstellungsprozesses, das in Figur 4f dargestellt ist, ist der erfindungsgemäße faseroptische Lichtleiter 1, bei dem sich die Lichtleitfasern 10 ohne Unterbrechung der Faserstruktur in den Verjüngungsbereich 12 fortsetzen. Das Hüllrohr 21 beziehungsweise dessen mit ausgezogener Bereich umgeben, als Hüllmaterial 20, den Verjüngungsbereich 12 und stabilisieren diesen. Dem Hüllrohr 21 kommt demnach mindestens eine Doppelfunktion zu, nämlich einmal die Lichtleiter 10 für den Herstellungsprozess zu einem Faserbündel zusammenzufassen und im Endprodukt als Hüllmaterial 20 den empfindlichen Übergangsbereich 12 am Ende des faseroptischen Lichtleiters 1 zu schützen. Optional ermöglicht das Hüllrohr 21, insofern es an der dem flexiblen Ende abgewandten Seite verschlossen ist, als weitere Funktion, Unterdruck während des Ausziehens des Übergangsbereichs 12 anlegbar zu machen und so eine weiter verbesserte Verschmelzung der Lichtleitfasern 10, bevorzugt auch mit dem Hüllrohr 21, insbesondere im Übergangsbereich 12 zu ermöglichen.

In weiteren Ausführungsformen kann, bei den zuvor beschriebenen Verfahrensschritten vorgesehen sein, dass das Hüllrohr 21 bereits einen unrunden Querschnitt aufweist und/ oder in einem vorgeschalteten Prozeß aus seinem an sich runden Querschnitt heraus in seinem Querschnitt unrund, insbesondere oval oder im wesentlichem D-förmig verformt wurde. In derartigen oder an sich unrunden Ausführungsformen wird als Durchmesser d1 beziehungsweise d2 die jeweils maximale Querabmessung verstanden.

Zur Herstellung einer bevorzugten Ausführungsform des erfindungsgemäßen faseroptischen Lichtleiters 1 werden beispielsweise zwischen 2200 bis 2500 Fasern flexible Kern-Mantel Lichtleitfasern 10 aus Glas mit dem Durchmesser 70 μm zu einem Faserbündel mit dem Durchmesser 3,80 mm bis 4,25 mm zusammengefasst und durch das beschriebene Verfahren ein faseroptischer Lichtleiter 1 mit einem Verjüngungsbereich der Länge $l_2$ von 15 bis 20 mm erzeugt, der ebenfalls von einem Glas als Hüllmaterial 20 umgeben ist, welches im Wesentlichen den gleichen thermischen Ausdehnungskoeffizienten wie das Kernglas der Lichtleitfasern 10 aufweist. Der Durchmesser $d_2$ 14 der zusammengeschmolzenen Lichtleitfasern 10 in der verjüngten Endfläche 13 des Verjüngungsbereichs 12 beträgt dabei 2,20 mm. $L_1$ kann beliebig gewählt werden, beträgt aber typischerweise einige zehn Zentimeter bis einige Meter, kann aber auch 10m Länge oder mehr erreichen. Die Endflächen 13, 15 können üblicherweise in Metallhülsen montiert und der Bereich zwischen den Metallhülsen mit einem flexiblen Schutzmantel aus Kunststoff umgeben werden. Die metallene Endhülse am Ende des Verjüngungsbereich 12 weist bevorzugt eine zylindrische Außengeometrie auf. Weil der Verjüngungsbereich 12 aber im Wesentlichen kegelförmig ist, hat diese Metallhülse daher bevorzugt eine dazu passende kegelförmige Innengeometrie, so dass eine im Wesentlichen formschlüssige Verbindung der Außenumfangsfläche des Übergangsbereichs 12 und der Innenumfangsfläche der Endhülse erzeugt werden kann. Von der Erfindung ist ebenfalls umfasst, wenn sich zwischen der Außenumfangsfläche des Übergangsbereichs 12 und der Innenumfangsfläche der Endhülse eine Schicht, beispielsweise eines Kunststoffes, insbesondere zum Beispiel eines Klebstoffs befindet.

Der erfindungsgemäße faseroptische Lichtleiter 1 weist durch die ununterbrochene Faserstruktur beim Übergang in den Verjüngungsbereich 12 gegenüber den aus dem Stand der Technik bekannten Lichtleitern eine verbesserte Transmission auf. Er ist durch das erfindungsgemäße Verfahren kostengünstig und aus Standardlichtleitfasern herstellbar. Dies macht seinen Herstellungsprozess flexibel und unterschiedlichste Geometrien und Durchmesser $d_1$ des Leitungsbereichs und $d_2$ des Verjüngungsbereichs 12 können je nach Bedarf rationell hergestellt werden.

Fig. 5a bis Fig. 5e zeigen schematisch, wie eine Verformung am starren Ende, also des vom Hüllmaterial 20 bzw, Hüllrohr 21 umschlossenen, fixierten Endes, des faseroptischen Lichtleiters 1 hergestellt werden kann.

Der faseroptische Lichtleiter 1 mit seinen Lichtleitfasern 10 und dem Hüllmaterial 20, welches zuvor, mit den Lichtleitfasern 10 verschmolzen wurde, in Fig. 5a dargestellt links als zylindrische Form mit konstanter Querschnittsfläche oder rechts dargestellt mit verjüngter Querschnittsfläche, werden mittels einer Heizeinrichtung 140 derart aufgeheizt (Fig. 5b), dass im nächsten Schritt (Fig. 5c) die eigentliche Umformung stattfinden kann. Hierbei wird mit Mitteln zum Umformen beziehungsweise Verformen, zum Beispiel mit einem Presswerkzeug oder Umformwerkzeug 150, die gewünschte Kontur in den faseroptischen Lichtleiter 1 eingepresst, also die gewünschte Kontur erzeugt, so dass die faseroptische Lichtleiter 1 gemäß Fig. 5d einen Umformbereich 18 aufweist. Dieser Umformbereich kann verschiedenen Ausprägungen haben, wie sie beispielhaft in den Figuren 3a bis 3i gezeigt sind.

Hierbei ist es möglich bzw. kann es notwendig sein, dass die Verformungen, insbesondere bei gewünschten beziehungsweise geforderten komplexen Ausführungsformen, in mehr als einem Verformungsschritt nacheinander, insbesondere auch mit unterschiedlichen Press- oder Umformwerkzeugen 150 an- oder aufgebracht werden. So kann beispielsweise zunächst eine Biegung am starren Enden des Lichtleiters 1 aus dessen ursprünglicher Längsachse 16 heraus erzeugt und anschließende eine weitere Biegung in insbesondere veränderter Ausrichtung, gefolgt von einer Umformung an der Endfläche 13 oder einem anderen Querschnittsbereich des starren Bereichs und/ oder gebogenen Endes. Dies kann auch mehrere Heizvorgänge umfassen, sowie entsprechende Verform- oder Umformprozesse geeignet gleichzeitig (parallel) und/ oder nacheinander (sequentiell) erfolgen.

Am Ende des Lichtleiters wird gegebenenfalls der überschüssige Teil des Hüllmaterials 20 abgetrennt (Fig. 5e), insbesondere wenn das Hüllmaterial 20 zuvor zu geschmolzen wurde. Daran schließt sich noch üblicherweise ein Schleif- und Polierprozess der Endfläche beziehungsweise der verjüngten Endfläche 13 an.

Bezugszeichenliste:

| | |
|---|---|
| 1 | Faseroptischer Lichtleiter |
| 10 | Lichtleitfaser |
| 11 | Durchmesser $d_1$ |
| 12 | Verjüngungsbereich |
| 13 | Verjüngte Endfläche |
| 14 | Durchmesser $d_2$ |
| 15 | Ursprüngliche Endfläche |
| 16 | Längsachse |
| 17 | Verformte Endfläche |
| 18 | Verformungsbereich |
| 19 | Übergangsbereich |
| | |
| 20 | Hüllmaterial |
| 21 | Hüllrohr |
| | |
| 100 | Unterdruckkammer |
| 110 | Pumpe |
| 120 | Heizeinrichtung |
| 130 | Trenneinrichtung |

(fortgesetzt)

| 140 | Heizeinrichtung |
|-----|-----------------|
| 150 | Presswerkzeug |
| | |
| | |
| | |
| | |

**Patentansprüche**

1. Faseroptischer Lichtleiter (1), beinhaltend eine Mehrzahl von Lichtleitfasern (10), welche als flexible Faserbündel mit im Wesentlichen konstantem Durchmesser $d_1$ (11) zusammengefasst beziehungsweise zusammenfassbar sind und an mindestens einem Ende des faseroptischen Lichtleiters (1) zumindest in Teilbereichen seiner Länge vollumfänglich von einem Hüllmaterial (20) umschlossen sind, wodurch ein starrer Abschnitt mit einer Längsachse (16) gebildet ist, und wobei die Lichtleitfasern (10) in dem starren Abschnitt miteinander und vorzugsweise auch mit dem Hüllmaterial (20) verschmolzen sind, und wobei
   der faseroptische Lichtleiter (1) im Bereich des Hüllmaterials (20) zumindest einmal aus seiner Längsachse (16) heraus gebogen und/oder die Querschnittsgeometrie des faseroptischen Lichtleiters (1) im Bereich des Hüllmaterials (20) zumindest einmal umgeformt ist.

2. Faseroptischer Lichtleiter (1) nach Anspruch 1, wobei der faseroptische Lichtleiter (1) einen flexiblen Bereich der Länge $l_1$ mit im Wesentlichen konstantem Durchmesser $d_1$ (11) an seiner ursprünglichen Endfläche (15) aufweist und zumindest einen sich daran anschließenden Verjüngungsbereich (12) der Länge $l_2$, in dem sich der Durchmesser des Lichtleiters (1) von dem Durchmesser $d_1$ (11) auf den Durchmesser $d_2$ (14) verringert, und wobei

   der Verjüngungsbereich (12) zumindest in Teilbereichen seiner Länge vollumfänglich von dem Hüllmaterial (20) umschlossen ist und sich die Faserstruktur ohne Unterbrechung von dem Bereich mit dem im Wesentlichen konstanten Durchmesser in den Verjüngungsbereich (12) fortsetzt, wobei
   der Verjüngungsbereich (12) eine verjüngte Endfläche (13) aufweist, in der die Lichtleitfasern (10) miteinander und vorzugsweise auch mit dem Hüllmaterial (20) verschmolzen sind.

3. Faseroptischer Lichtleiter (1) nach Anspruch 1 oder 2, wobei die Querschnittsfläche der Endfläche am Lichtleiter (1) oder die verjüngte Endfläche (13) des verjüngten Lichtleiters (1) im Wesentlichen unrund, oval, D-förmig, nierenförmig oder polygonal verformt ist.

4. Faseroptischer Lichtleiter (1) nach zumindest einem der vorhergehenden Ansprüche, wobei

   die Lichtleitfasern (10) Kern-Mantel-Fasern aus Glas sind und
   das Hüllmaterial (20) aus einem Glas besteht, dessen thermischer Ausdehnungskoeffizient sich höchstens um 50%, bevorzugt höchsten um 30% von dem thermischen Ausdehnungskoeffizienten des Kernglases der Lichtleitfasern (10) unterscheidet.

5. Faseroptischer Lichtleiter (1) nach Anspruch 4, **gekennzeichnet durch** zumindest eines der folgenden Merkmale

   - das Hüllmaterial (20) umfasst ein farbiges Glas, bevorzugt ein Schwarz- oder Braunglas
   - das Hüllmaterial (20) umfasst ein einfärbbares Glas, insbesondere schwarz oder braun einfärbbares Glas,
   - das Hüllmaterial (20) umfasst ein licht-absorbierend beschichtetes Glas.

6. Faseroptischer Lichtleiter (1) nach Anspruch 4, wobei
   die Lichtleitfasern (10) aus einem Pb-freien Glassystem für Kern und Mantel bestehen und die Lichtleitfasern (10) eine numerische Apertur NA von mind. 0,50, bevorzugt von mind. 0,60 und besonders bevorzugt von mind. 0,80 aufweisen.

7. Faseroptischer Lichtleiter (1) nach zumindest einem der vorhergehenden Ansprüche, wobei

   der Durchmesser $d_1$ (11) im Bereich von 0,5 mm bis 3,0 mm, bevorzugt im Bereich von 2,5 mm bis 3,0 mm bei einem Durchmesser der Lichtleitfasern (10) von $(30 \pm 4)$ $\mu$m beträgt oder wobei
   der Durchmesser $d_1$ (11) im Bereich von 0,5 mm bis 8,0 mm, bevorzugt im Bereich von 2,5 mm bis 6,5 mm bei einem Durchmesser der Lichtleitfasern (10) von $(50 \pm 4)$ $\mu$m oder $(70 \pm 4)$ $\mu$m beträgt.

8. Faseroptischer Lichtleiter (1) nach zumindest einem der vorhergehenden Ansprüche, wobei
   die Dicke des Hüllmaterials (20) im Bereich von 0,1 mm bis 0,5 mm, bevorzugt im Bereich von 0,15 mm bis 0,25 mm beträgt.

9. Faseroptischer Lichtleiter (1) nach zumindest einem der Ansprüche 4 bis 8, wobei

   das Hüllmaterial (20) als Glashülse ausgebildet ist und wobei
   die Glashülse an der Seite, welche zum flexiblen Teil des Lichtleiters (1) mit den Lichtleitfasern (10) hinweist, einen ausgebildeten Kragen oder einen innen liegenden Konus aufweist.

10. Faseroptischer Lichtleiter (1) nach zumindest einem der vorhergehenden Ansprüche, wobei
    zumindest das verformte und/ oder verjüngte Ende des faseroptischen Lichtleiters (1) mit der verjüngten Endfläche (13) am Verjüngungsbereich (12) und das andere Ende mit der ursprünglichen Endfläche (15), die sich an den Bereich mit dem im Wesentlichen konstanten Durchmesser $d_1$ (11) anschließt, in jeweils einer Endhülse aus Metall oder Kunststoff montiert sind und sich zwischen dem faseroptischen Lichtleiter (1) und der Innenseite der Endhülsen zumindest bereichsweise eine Schicht aus Kunststoff befindet.

11. Verwendung des faseroptischen Lichtleiters nach einem der Ansprüche 1 bis 10 in einem medizintechnischen oder industriellen Endoskop-Instrument und/ oder als Lichtleiter zwischen einer Lichtquelle und dem Endoskop-Instrument oder einer Analyse-Einrichtung oder als Lichtleiter zwischen einem Behälter und einer Detektor-Einheit.

12. Verfahren zum Herstellen eines faseroptischen Lichtleiters (1), welcher eine Mehrzahl von Lichtleitfasern (10) aufweist, welche als flexible Faserbündel mit im Wesentlichen konstantem Durchmesser $d_1$ (11) zusammengefasst beziehungsweise zusammenfassbar sind, umfassend die Verfahrensschritte

    - Einbringen der Lichtleitfasern 10 als Faserbündel in ein Hüllrohr (21), welches das Faserbündel auf einer Teillänge umschließt,
    - Erwärmen des in dem Hüllrohr (21) befindlichen Faserbündels und des Hüllrohres (21) auf zumindest einer Teillänge des Hüllrohres bis mindestens zum Erweichen der Lichtleitfasern (10) und des Hüllrohres (21),
    - Kollabieren des Hüllrohres (21) auf das Faserbündel der Lichtleitfasern (10)
    - Verformen des Lichtleiters (1) im Bereich des Hüllrohres (21) aus seiner Längsachse (16) heraus und/ oder Umformen hinsichtlich seiner Querschnittsgeometrie.

13. Verfahren nach Anspruch 11, welches nach dem Erwärmen der in dem Hüllrohr (21) befindlichen Faserbündels und des Hüllrohres (21) zusätzlich die Verfahrensschritte

    - Ausziehen des erwärmten Bereichs, bis das in dem Hüllrohr (21) befindliche Faserbündel zusammen mit dem Hüllmaterial (20) in zumindest einem Teilbereich den Durchmesser $d_2$ (14) aufweist und so ein Verjüngungsbereich (12) gebildet wird, in dem die Faserstruktur nicht unterbrochen wird,
    - Abtrennen des Hüllrohres (21) und des darin befindlichen Faserbündels im Verjüngungsbereich (12) an der Stelle des Durchmessers $d_2$ aufweist.

14. Verfahren nach einem der Ansprüche 12 oder 13, wobei
    das Hüllrohr (21) einen unrunden, bevorzugt ovalen oder im wesentlichem D-förmigen Querschnitt aufweist.

15. Verfahren nach einem der Ansprüche 12 bis 14, wobei das Hüllrohr (21) einseitig verschlossen wird und vor dem Verformen oder dem Ausziehen des erwärmten Bereichs Unterdruck an das Hüllrohr (21) und die Lichtleitfasern (10) angelegt wird, so dass die Lichtleitfasern (10) miteinander, vorzugsweise auch mit dem Hüllrohr (21), verschmelzen.

16. Verfahren nach zumindest einem der Ansprüche 12 bis 15, wobei der erwärmte Bereich vor dem Abtrennen des Hüllrohres (21) und des darin befindlichen Faserbündels abgekühlt wird.

17. Verfahren nach zumindest einem der Ansprüche 12 bis 16, wobei die Endfläche oder die den abgetrennten Verjüngungsbereich (12) abschließende verjüngte Endfläche (13) geschliffen und/oder poliert wird.

Fig. 1

Fig. 2

Fig. 3a

Fig. 3b

Fig. 3c

Fig. 3d

Fig. 3e

Fig. 3f

Fig. 3g

Fig. 3h

Fig. 3i

Fig. 4a

Fig. 4b

Fig. 4c

Fig. 4d

Fig. 4e

Fig. 4f

Fig. 5a

Fig. 5b

Fig. 5c

Fig. 5d

Fig. 5e

Europäisches
Patentamt

European
Patent Office

Office européen
des brevets

# EUROPÄISCHER RECHERCHENBERICHT

**Nummer der Anmeldung**

**EP 23 16 0638**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2006/045444 A1 (MILLER WILLIAM J [US] ET AL) 2. März 2006 (2006-03-02) * Abbildungen 4,6,9 * * Absatz [0004] * * Absatz [0039] * * Absätze [0043] - [0045] * * Absätze [0048] - [0049] * * Absatz [0056] * ----- | 1-17 | INV. G02B6/04 A61B1/07 |
| X | US 2015/016140 A1 (WEINGAERTNER THOMAS [DE] ET AL) 15. Januar 2015 (2015-01-15) * Abbildungen 1-4,6 * * Absatz [0043] * * Absatz [0052] * * Absatz [0080] * * Absatz [0076] * ----- | 1-17 | |
| X | DE 198 30 335 A1 (SCHMIDT ES BENDER HUNGARIA KFT [HR]) 14. Januar 1999 (1999-01-14) * Abbildungen 4,5 * * Anspruch 1 * * Sp. 1, Z. 19-22 * ----- | 1-17 | RECHERCHIERTE SACHGEBIETE (IPC)  G02B |
| X | DE 37 44 367 C1 (SCHOTT GLASWERKE) 24. August 1989 (1989-08-24) * Abbildung 2 * * Sp. 1, Z. 9-19 * * Sp. 3, letzter Paragraph * ----- | 1-17 | |

Der vorliegende Recherchenbericht wurde für alle Patentansprüche erstellt

| Recherchenort | Abschlußdatum der Recherche | Prüfer |
|---|---|---|
| Den Haag | 2. August 2023 | Dregely, Daniel |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 23 16 0638

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-08-2023

| Im Recherchenbericht angeführtes Patentdokument | Datum der Veröffentlichung | Mitglied(er) der Patentfamilie | Datum der Veröffentlichung |
|---|---|---|---|
| US 2006045444 A1 | 02-03-2006 | EP 1784667 A2 | 16-05-2007 |
| | | JP 4969447 B2 | 04-07-2012 |
| | | JP 2008511871 A | 17-04-2008 |
| | | US 2006045444 A1 | 02-03-2006 |
| | | WO 2006026542 A2 | 09-03-2006 |
| US 2015016140 A1 | 15-01-2015 | CH 708055 A2 | 14-11-2014 |
| | | DE 102013208838 A1 | 20-11-2014 |
| | | FR 3005653 A1 | 21-11-2014 |
| | | JP 2014223310 A | 04-12-2014 |
| | | US 2015016140 A1 | 15-01-2015 |
| DE 19830335 A1 | 14-01-1999 | DE 19830335 A1 | 14-01-1999 |
| | | HU 9701159 A2 | 28-07-1999 |
| DE 3744367 C1 | 24-08-1989 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10013482 C2
- DE 19703515 C1
- US 11034612 B2